(19) Europäisches Patentamt / European Patent Office / Office européen des brevets

(11) **EP 3 664 768 B1**

(12) **EUROPEAN PATENT SPECIFICATION**

(45) Date of publication and mention
of the grant of the patent:
**28.02.2024 Bulletin 2024/09**

(21) Application number: **18759363.7**

(22) Date of filing: **10.08.2018**

(51) International Patent Classification (IPC):
*A61K 8/06* *(2006.01)*    *A61K 8/39* *(2006.01)*
*A61K 8/86* *(2006.01)*    *A61Q 5/04* *(2006.01)*
*A61Q 5/12* *(2006.01)*    *A61Q 9/04* *(2006.01)*
*A61Q 15/00* *(2006.01)*    *A61Q 19/00* *(2006.01)*
*A61Q 19/04* *(2006.01)*    *C11D 1/825* *(2006.01)*
*C11D 1/72* *(2006.01)*    *C11D 1/722* *(2006.01)*

(52) Cooperative Patent Classification (CPC):
**A61Q 19/008; A61K 8/06; A61K 8/39; A61K 8/86;
A61Q 5/04; A61Q 5/12; A61Q 9/04; A61Q 15/00;
A61Q 19/04;** A61K 2800/5422; A61K 2800/596

(86) International application number:
**PCT/GB2018/052285**

(87) International publication number:
**WO 2019/030536 (14.02.2019 Gazette 2019/07)**

(54) **EMULSIFIER SYSTEM, PERSONAL CARE PRODUCT, METHOD AND USE**

EMULGATORSYSTEM, KÖRPERPFLEGEPRODUKT, VERFAHREN UND VERWENDUNG

SYSTÈME ÉMULSIFIANT, PRODUIT D'HYGIÈNE CORPORELLE, PROCÉDÉ ET UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.08.2017 GB 201712844**

(43) Date of publication of application:
**17.06.2020 Bulletin 2020/25**

(73) Proprietor: **Croda International PLC
Goole, Yorkshire DN14 9AA (GB)**

(72) Inventors:
• **BÉNARD, Elodie
Goole
Yorkshire DN14 9AA (GB)**
• **CHAVAN, Bhaven
Goole
Yorkshire DN14 9AA (GB)**
• **ROUSE, Sean Philip Nigel
Goole
Yorkshire DN14 9AA (GB)**

(74) Representative: **Craven, Ian et al
Croda Europe Limited
Intellectual Property Department
Cowick Hall
Snaith
Goole Yorkshire DN14 9AA (GB)**

(56) References cited:
DE-A1- 4 122 470    US-A1- 2001 014 654
US-A1- 2006 280 710

**Description**

**Field of Invention**

The present invention relates to a composition, an emulsifier system comprising the composition, a personal care product comprising the emulsifier system, a method of emulsifying using the composition and use of the composition as an emulsifier. In particular, the invention relates to a composition comprising an emulsifier system operable to emulsify formulations stably under extreme conditions, for example formulations with a high salt content or a low or high pH, or formulations requiring stability in extreme circumstances such as those subjected to high temperatures for sustained periods of time.

**Background**

Alkoxylated esters have been used for many years as surface active agents (or surfactants), having emulsifying, dispersing, wetting and/or solubilising properties in a wide range of applications such as personal care, home care, industrial, food, and many others. In particular, alkoxylated esters have been used as emulsifiers in personal care applications, for example skin care, sunscreens, toiletries, decorative cosmetics, perfumes and fragrances. DE4122470 discloses an aqueous cleaning hands, hard surfaces and glass containing surfactant, co-polymer, phosphate, white spirit and reducing or oxidising agent.

Current commercially available alkoxylated esters are effective emulsifiers in many applications, but there is still a requirement to improve the properties of emulsifiers, particularly in personal care applications. These properties may include the flexibility of use of the emulsifier in different systems and the ability to emulsify in strenuous conditions such as a high internal phase content, a high or low pH or long term storage at an elevated temperature. An example of a further desirable property of an emulsifier would be if the emulsifier could be used in a particular system without the need for a co-emulsifier.

**Summary of the Invention**

It is an object of the present invention to address at least one of the above or other disadvantages associated with the prior art.

According to a first aspect of the present invention, there is provided a composition comprising:

a) an alkoxylated fatty alcohol compound of formula (A)

$$R^1(OC_2H_4)_n(OC_3H_6)_m \qquad (A)$$

wherein $R^1$ is a $C_{16}$-$C_{26}$ alkyl or alkenyl group,
n is the mean number of moles of $-(OC_2H_4)-$ per molecule of formula (A) present in the compound and is between 15 and 60,
m is the mean number of moles of $-(OC_3H_6)-$ per molecule of formula (A) present in the compound and is between 0 and 20,
and n+m > 15 ; and

b) an alkoxylated fatty alcohol compound of formula (B)

$$R^2(OC_2H_4)_r(OC_3H_6)_s \qquad (B)$$

wherein $R^2$ is a $C_{16}$-$C_{26}$ alkyl or alkenyl group,

r is the mean number of moles of $-(OC_2H_4)-$ per molecule of formula (B) present in the compound and is between 0.01 and 14.99,

s is the mean number of moles of $-(OC_3H_6)-$ per molecule of formula (B) present in the compound and is between 0.01 and 14.99, and

$$r+s \leq 15.$$

According to a second aspect of the present invention, there is provided an emulsifier system comprising a composition according to the first aspect.

According to a third aspect of the present invention, there is provided a personal care formulation comprising a composition according to the first aspect or an emulsifier system according to the second aspect.

According to a fourth aspect of the present invention, there is provided a method of stabilising an emulsion as defined in the claims.

According to a fifth aspect of the present invention, there is provided the use of a composition, or an emulsifier system as defined in the claims.

All of the features described herein may be combined with any of the above aspects, in any combination.

The present invention is based in part on the recognition by the inventors that a composition of the first aspect of the invention has advantageous properties due to its particular combination of components.

At room temperature, formulations comprising high concentrations of salts or having low or high pHs will be under significant stress. Traditional emulsifiers, being non-ionic, will have a reduced cloud point due to the salt concentration of the formulation, hence reducing the HLB of the emulsifiers and the overall system making them more oil soluble. This leads traditional emulsifiers to sit further in the oil phase of an emulsion, and less at the interface. When the temperature of the formulation is increased to 60°C, it is further stressed and the cloud point and HLB will be further reduced, thus pushing the emulsifiers further into the oil phase and far enough away from the interface to cause instability to be seen. Furthermore, where traditional emulsifiers are only EO based, the EO chain can curl up and start to bond with itself in preference to the water phase, thus leading to further instability.

Without being bound by theory, the composition and/or emulsifier system of the present invention provides a starting material that has a higher cloud point. Increasing the temperature and also having salt in the formulation will reduce the cloud point and HLB but, with the aid of the composition and/or emulsifier system according to the invention, not to the same degree as formulations using emulsifier systems according to the prior art. This gives the products according to the present invention a distinct advantage. Having a combination of fatty alcohols with different length alkoxylated chains, particularly the fatty alcohol with the longer alkoxylated chain, in the composition / emulsifier system also adds further stability since it provides a composition / emulsifier system with a good amount of hydrophilic and lipophilic stabilisation. The inclusion of PO at the end of the EO chain will help to prevent the EO chain curling thus helping to stabilise the system further. In addition, PO likes to sit in the oil phase of an emulsion, but also can have a tendency to sit in the water phase. Therefore pushing of the emulsifier into different emulsion phases would not affect the system as much compared to an EO only system.

## Detailed description of the Invention

It will be understood that any upper or lower quantity or range limit used herein may be independently combined.

It will be understood that, when describing the number of carbon atoms in a substituent group (e.g. '$C_1$ to $C_6$'), the number refers to the total number of carbon atoms present in the substituent group, including any present in any branched groups. Additionally, when describing the number of carbon atoms in, for example fatty acids, this refers to the total number of carbon atoms including the one at the carboxylic acid, and any present in any branch groups.

Many of the chemicals which may be used to produce the composition of the present invention are obtained from natural sources. Such chemicals typically include a mixture of chemical species due to their natural origin. Due to the presence of such mixtures, various parameters defined herein can be an average value and may be non-integral.

The term 'residue' as used herein is the part of a reactant molecule which remains in the reaction product compound after a reaction has occurred.

The term 'alkyl' is well known in the art and, when used herein, means a saturated $C_{16}$ to $C_{26}$, preferably $C_{16}$ to $C_{24}$, more preferably $C_{18}$ to $C_{24}$ group, and includes cetyl, stearyl, arachidyl, behenyl, lignceryl and cerotinyl groups. Unless otherwise specified, alkyl groups may be linear or branched (particularly preferred branched groups include methyl, ethyl, propyl, butyl, t-butyl and isopropyl), be cyclic, acyclic or part cyclic/acyclic, and/or be unsubstituted, substituted, terminated or interrupted by one or more substituents selected from halogen-, nitrogen- and/or oxygen-containing groups.

The term 'alkenyl' is also well known in the art and, when used herein, means an unsaturated $C_{16}$ to $C_{26}$, preferably $C_{16}$ to $C_{24}$, more preferably $C_{18}$ to $C_{24}$ group, and includes oleyl groups. Unless otherwise specified, alkenyl groups may be linear or branched (particularly preferred branched groups include methyl, ethyl, propyl, butyl, t-butyl and isopropyl), be cyclic, acyclic or part cyclic/acyclic, and/or be unsubstituted, substituted, terminated or interrupted by one or more substituents selected from halogen-, nitrogen- and/or oxygen-containing groups.

## The Alkoxylated Fatty Alcohols

The term 'fatty alcohol' as used herein means long-chain primary alcohols, preferably with a chain length of 16 to 26 carbon atoms, which may be derived from natural sources such as fats and oils. Preferably, the fatty alcohols used to produce the alkoxylated fatty alcohols of the present invention are derived from vegetable sources, although it is also possible to derive the alcohols from animal sources or petrochemical sources. The alcohols used in the present invention will normally be obtained from naturally occurring esters by hydrogenation.

The fatty alcohol used in the present invention may be a mixture of alcohols of different chain lengths. In this case, the mixture may be a commercially available mixture, or may be made from a combination of different chain length alcohols.

The alcohol used in the present invention may be selected from the group comprising cetyl alcohol, cetostearyl alcohol, palmitoleyl alcohol, stearyl alcohol, isostearyl alcohol, oleyl alcohol, arachidyl alcohol, behenyl alcohol, isobehenyl alcohol, erucyl alcohol, lignceryl alcohol and cerotinyl alcohol. Preferably, the alcohol used in the present invention is selected from cetyl alcohol, cetostearyl alcohol, stearyl alcohol, isostearyl alcohol, arachidyl alcohol, behenyl alcohol, isobehenyl alcohol, lignceryl alcohol and cerotinyl alcohol. More preferably, the alcohol used in the present invention is selected from stearyl alcohol, isostearyl alcohol, behenyl alcohol and isobehenyl alcohol, most preferably from stearyl alcohol and behenyl alcohol, desirably behenyl alcohol.

Preferably, $R^1$ is the residue of a fatty alcohol, or mixture of fatty alcohols, as defined above.

Preferably, $R^2$ is the residue of a fatty alcohol, or mixture of fatty alcohols, as defined above.

The alkylene oxides used in the present invention are preferably ethylene oxide and propylene oxide. These oxides are readily available to the skilled person and can be derived from petrochemical sources (eg by the oxidation of ethylene or propylene obtained from hydrocarbon cracking) or from natural sources (eg by the oxidation of ethylene obtained from bioethanol from biomass).

Preferably, $-(OC_2H_4)-$ is the residue of ethylene oxide as defined above.

Preferably, $-(OC_3H_6)-$ is the residue of propylene oxide as defined above.

In the alkoxylated fatty alcohol compound of formula (A), preferably, $R^1$ is a $C_{16}$-$C_{26}$ alkyl or alkenyl group, preferably a $C_{16}$-$C_{24}$ alkyl or alkenyl group, preferably a $C_{18}$-$C_{24}$ alkyl or alkenyl group, more preferably a $C_{18}$-$C_{22}$ alkyl or alkenyl group, most preferably a $C_{22}$ alkyl or alkenyl group.

Preferably, $R^1$ is an alkyl group. $R^1$ may be branched or linear, preferably linear. $R^1$ may be cyclic or acyclic, preferably acyclic. $R^1$ may be present as a mixture of alkyl or alkenyl groups.

n is the mean number of moles of $-(OC_2H_4)-$ per molecule of formula (A) present in the compound and is between 15 and 60, preferably between 15 and 50, more preferably between 22 and 40, most preferably between 24 and 34.

m is the mean number of moles of $-(OC_3H_6)-$ per molecule of formula (A) present in the compound and is between 0 and 20, preferably between 0 and 10, more preferably between 0 and 5. Most preferably, m is 0.

n+m is the mean total number of moles of $-(OC_2H_4)-$ and $-(OC_3H_6)-$ per molecule of formula (A) present in the compound and is greater than 15, preferably greater than 22 and most preferably greater than 25. When m = 0, n+m = n.

Preferably, the compound of formula (A) has the formula (A')

$$R^1(OC_2H_4)_n \qquad (A')$$

In the alkoxylated fatty alcohol compound of formula (B), preferably, $R^2$ is a $C_{16}$-$C_{26}$ alkyl or alkenyl group, preferably a $C_{16}$-$C_{24}$ alkyl or alkenyl group, preferably a $C_{18}$-$C_{24}$ alkyl or alkenyl group, more preferably a $C_{18}$-$C_{22}$ alkyl or alkenyl group, most preferably a $C_{22}$ alkyl or alkenyl group.

Preferably, $R^2$ is an alkyl group. $R^2$ may be branched or linear, preferably linear. $R^2$ may be cyclic or acyclic, preferably acyclic. $R^2$ may be present as a mixture of alkyl or alkenyl groups.

Preferably, $R^2$ is the same as $R^1$. Alternatively, $R^2$ may be different to $R^1$.

r is the mean number of moles of $-(OC_2H_4)-$ per molecule of formula (B) present in the compound and is between 0.01 and 14.99, preferably between 0.1 and 10, more preferably between 0.5 and 5, and most preferably between 1 and 4.

s is the mean number of moles of $-(OC_3H_6)-$ per molecule of formula (B) present in the compound and is between 0.01 and 14.99, preferably between 0.05 and 10, more preferably between 0.05 and 5, more preferably between 0.1 and 1, most preferably between 0.1 and 0.6.

r+s is the mean total number of moles of $-(OC_2H_4)-$ and $-(OC_3H_6)-$ per molecule of formula (B) present in the compound and is less than 15, preferably less than 10 and most preferably less than 6.

The compound of formula (B) may be a mixture of two or more components.

Preferably, the compound of formula (B) may be a mixture of components (B') and (B") having the formulas:

$$R^2(OC_2H_4)_r \qquad (B')$$

4

and

$$R^2(OC_2H_4)_r(OC_3H_6)_t \qquad (B'')$$

, respectively, wherein $R^2$ and r are as described above and t is the mean number of moles of - $(OC_3H_6)$ per molecule of formula (B'') present in the compound and is between 0.01 and 14.99, preferably between 0.05 and 10, more preferably between 0.1 and 5, most preferably between 0.5 and 3.

When present as a mixture of components (B') and (B''), the compound of formula (B) preferably has a mean number of moles of propylene oxide per molecule (ie the mean of the total ethoxylated alcohol and ethoxy- and propoxylated alcohol components) which is equal to s.

The composition of the present invention optionally further comprises an alkoxylated fatty alcohol compound of formula (C)

$$R^3(OC_2H_4)_x(OC_3H_6)_y \qquad (C)$$

wherein $R^3$ is a $C_{16}$-$C_{26}$ alkyl or alkenyl group,
x is the mean number of moles of -$(OC_2H_4)$- per molecule of formula (C) present in the compound and is between 0.01 and 14.99,
y is the mean number of moles of -$(OC_3H_6)$- per molecule of formula (C) present in the compound and is between 0.01 and 14.99, and

$$x+y \leq 15 \neq r+s.$$

In the alkoxylated fatty alcohol compound of formula (C), preferably, $R^3$ is a $C_{16}$-$C_{26}$ alkyl or alkenyl group, preferably a $C_{16}$-$C_{24}$ alkyl or alkenyl group, preferably a $C_{18}$-$C_{24}$ alkyl or alkenyl group, more preferably a $C_{18}$-$C_{22}$ alkyl or alkenyl group, most preferably a $C_{22}$ alkyl or alkenyl group.

Preferably, $R^3$ is an alkyl group. $R^3$ may be branched or linear, preferably linear. $R^3$ may be cyclic or acyclic, preferably acyclic. $R^3$ may be present as a mixture of alkyl or alkenyl groups.

Preferably, $R^3$ is the residue of a fatty alcohol, or mixture of fatty alcohols, as defined above.

Preferably, $R^3$ is the same as $R^2$. Alternatively, $R^3$ may be different to $R^2$.

Preferably, $R^3$ is the same as $R^1$. Alternatively, $R^3$ may be different to $R^1$.

Preferably, x is the mean number of moles of -$(OC_2H_4)$- per molecule of formula (C) present in the compound and is between 0.01 and 14.99, preferably between 0.1 and 10, more preferably between 0.5 and 5, and most preferably between 1 and 4. Preferably, x is different to r.

Preferably, y is the mean number of moles of -$(OC_3H_6)$- per molecule of formula (C) present in the compound and is between 0.01 and 14.99, preferably between 0.05 and 10, more preferably between 0.05 and 5, more preferably between 0.1 and 1, most preferably between 0.1 and 0.6. y may be the same as or different to s.

Preferably x+y is mean total number of moles of -$(OC_2H_4)$- and -$(OC_3H_6)$- per molecule of formula (B) present in the compound and is less than 15, preferably less than 10 and most preferably less than 6. Preferably, x+y is different to r+s.

The compound of formula (C) may be a mixture of two or more components. Preferably, the compound of formula (C) may be a mixture of components (C') and (C'') having the formulas:

$$R^3(OC_2H_4)_x \qquad (C')$$

and

$$R^3(OC_2H_4)_x(OC_3H_6)_z \qquad (C'')$$

, respectively, and
wherein $R^3$ and x are as described above and z is the mean number of moles of -$(OC_3H_6)$- per molecule of formula (C'') present in the compound and is between 0.01 and 14.99, preferably between 0.05 and 10, more preferably between 0.1 and 5, most preferably between 0.5 and 3. z may be the same as or different to t.

When present as a mixture of components (C') and (C''), the compound of formula (C) preferably has a mean number of moles of propylene oxide per molecule (ie the mean of the total ethoxylated alcohol and ethoxy- and propoxylated alcohol components) which is equal to y.

Reaction Method

Alkoxylated fatty alcohols are known in the art. As such, their production would be well known to the skilled person.

The alkoxylated fatty alcohols of the invention may be produced in a conventional alkoxylation reaction, for example by reacting a fatty alcohol with ethylene oxide and, where applicable, propylene oxide. The reaction may occur in one stage, i.e. where all of the reactants are mixed together and reacted, or in two stages, e.g. where the fatty alcohol is mixed with the ethylene oxide and reacted together, followed by subsequent addition and reaction of the propylene oxide where required. Preferably the reaction occurs in two stages.

The Composition

The composition comprises:

a) an alkoxylated fatty alcohol component of formula (A);
b) an alkoxylated fatty alcohol component of formula (B); and
c) optionally, an alkoxylated fatty alcohol component of formula (C).

In one embodiment, the composition consists essentially of:

a) an alkoxylated fatty alcohol component of formula (A);
b) an alkoxylated fatty alcohol component of formula (B); and
c) an alkoxylated fatty alcohol component of formula (C).

Preferably, the composition is an emulsifier system.

The ratios of components may advantageously influence the properties of the composition.

Preferably, the molar amount of component b) in the composition is equal to or greater than the molar amount of component a).

Preferably the molar amount of b) in the composition is equal to or greater than the molar amount of c), when present.

The mean number of moles of component b) to component a) is suitably in the range from 0.1 to 8:1, preferably 0.5 to 6:1, more preferably 1 to 5:1, particularly 2 to 4:1, and especially 2.8 to 3.5:1.

The mean number of moles of component b) to component c), where present, is suitably in the range from 0.1 to 10:1, preferably 0.5 to 8:1, more preferably 1 to 6:1, particularly 2 to 5:1, and especially 3.5 to 4.5:1.

The mean number of moles of component a) to component c), where present, is suitably in the range from 0.1 to 5:1, preferably 0.4 to 3:1, more preferably 0.6 to 2.5:1, particularly 0.8 to 2:1, and especially 0.9 to 1.5:1.

Preferably, the composition is anhydrous. By the use of the term anhydrous herein, it is meant that the blend preferably comprises a maximum of 10% by weight water. More preferably, the blend comprises a maximum of 7% by weight water, most preferably, 5% and desirably 2% by weight. Preferably, the blend comprises 0.01% to 10% by weight water, preferably 0.05% to 5%, most preferably 0.1% to 2% by weight.

The composition of the invention may be used to stabilise an emulsion. The composition may be an emulsifier system.

The composition of the invention may have the properties of a surfactant, emulsifier, dispersant, stabiliser, solubiliser, pigment wetter and/or rheology modifier. The invention also includes the use of the composition as a surfactant, emulsifier, dispersant, stabilizer, solubiliser, pigment wetter and/or rheology modifier, preferably as a surfactant and/or emulsifier, more preferably as an emulsifier.

The composition may have an acid value (measured as described herein) in the range from 0 to 15, preferably 0 to 10, more preferably 0 to 5, particularly 0 to 2, and especially 0 to 1 mgKOH/g.

The composition may have a hydroxyl (OH) value (measured as described herein) in the range from 50 to 300, preferably 70 to 220, more preferably 80 to 190, particularly 90 to 175, and especially 100 to 165 mgKOH/g.

The Emulsifier System

The composition of the invention is suitable for use in forming emulsions (and dispersions), i.e. as the, or as part of the, emulsifier system. The emulsion may be a water in oil emulsion, oil in polyol (e.g. glycerol) emulsion or oil in water emulsion. The emulsion may be a multiple emulsion, for example a water in oil in water emulsion.

The emulsifier system may be added into the water phase or the oil phase of an emulsion. Alternatively, the components of the emulsifier system may be added into different phases of the emulsion, for example, component a) may be added into the water phase whilst component b), and component c) when present, are added into the oil phase, or vice versa, component a) may be added into the oil phase whilst component b), and component c) when present, are added into the water phase.

The emulsion is preferably for use in a personal care formulation, more preferably a skin care product for example a sunscreen, cosmetic, antiperspirant, depilatory or dermatological product, or a hair care product for example a shampoo, conditioner, hair dye or hair relaxer product.

The composition of the invention is particularly suitable for the preparation of water-in-oil emulsions which contain a high concentration of salts, have a high or low pH, or require stability at elevated temperatures.

The composition of the invention may be a high performance water in oil emulsifier. It may be used at a lower wt % inclusion level than a comparative emulsifier to provide an equivalent level of emulsion stability.

The oil phase of the emulsion preferably comprises an emollient oil of the type used in a personal care formulation. The emollient is preferably an oily material which is liquid at ambient temperature (i.e. about 23°C). Alternatively it can be solid at ambient temperature, in which case in bulk it will usually be a waxy solid, provided it is liquid at an elevated temperature at which it can be included in and emulsified in the composition. The manufacture of the formulation preferably uses temperatures up to 100ºC, more preferably about 80ºC, and therefore such solid emollients will preferably have melting temperatures of less than 100ºC, and more preferably less than 70ºC. The emulsifier may be used cold process or using a semi-hot process if required.

The oil phase of the emulsion may comprise at least one ester oil, vegetable oil, alcohol, paraffin oil or silicone.

Suitable oil phase components include non-polar oils, for example mineral or paraffin, especially isoparaffin, oils, such as that sold by Croda as Arlamol (trade mark) HD; or medium polarity oils, for example vegetable ester oils such as jojoba oil, vegetable glyceride oils, animal glyceride oils, such as that sold by Croda as Crodamol (trade mark) GTCC (caprylic/capric triglyceride), synthetic oils, for example synthetic ester oils, such as isopropyl palmitate and those sold by Croda as Crodamol IPP and Arlamol DOA, ether oils, particularly of two fatty e.g. C8 to C18 alkyl residues, such as that sold by Cognis as Cetiol OE (dicaprylether), guerbet alcohols such as that sold by Cognis as Eutanol G (octyl dodecanol), or silicone oils, such as dimethicone oil such as those sold by Dow Corning as Xiameter PMX-200, cyclomethicone oil, or silicones having polyoxyalkylene side chains to improve their hydrophilicity; or highly polar oils including alkoxylate emollients for example fatty alcohol propoxylates such as that sold by Croda as Arlamol PS15E (propoxylated stearyl alcohol). Suitable emollient materials that can be solid at ambient temperature but liquid at temperatures typically used to make the formulations of this invention include jojoba wax, tallow and coconut wax/oil. When non-polar oils are used it may be desirable to use relatively high concentrations of the composition according to the present invention, in order to achieve suitably satisfactory emulsification, particularly to obtain small oil droplets.

Mixtures of emollients can and often will be used, and in some cases solid emollients may dissolve wholly or partly in liquid emollients or in combination the freezing point of the mixture is suitably low. Where the emollient composition is a solid (such as fatty alcohols) at ambient temperature, the resulting dispersion may technically not be an emulsion (although in most cases the precise phase of the oily disperse phase cannot readily be determined) but such dispersions behave as if they were true emulsions and the term emulsion is used herein to include such compositions.

The concentration of the oil phase may vary widely. The amount of oil in the emulsion is suitably in the range from 1 to 90%, preferably 3 to 60%, more preferably 5 to 40%, particularly 8 to 20%, and especially 10 to 15% by weight of the total formulation.

The amount of water (or polyol, e.g. glycerin) present in the emulsion is suitably greater than 5%, preferably in the range from 30 to 90%, more preferably 50 to 90%, particularly 70 to 85%, and especially 75 to 80% by weight of the total formulation.

The amount of the composition or emulsifier system of the invention in an emulsion or personal care formulation according to the present invention may be at least 0.1%, preferably at least 0.5%, more preferably at least 1%, particularly preferably at least 1.5%, and especially preferably at least 2%, by weight of the total formulation.

The amount of the composition or emulsifier system of the invention in an emulsion or personal care formulation according to the present invention may be at most 10%, preferably at most 8%, more preferably at most 7%, particularly preferably at most 6%, and especially preferably at most 5.5%, by weight of the total formulation.

The amount of the composition or emulsifier system of the invention in an emulsion or personal care formulation according to the present invention is suitably in the range from 0.1 to 10%, preferably 0.5 to 8%, more preferably 1 to 7%, particularly 1.5 to 6%, and especially 2 to 5.5%, by weight of the total formulation.

The emulsions according to the present invention may also contain other additional surfactant materials which form part of the emulsifier system. Other suitable surfactants include relatively hydrophilic surfactants, e.g. having a HLB value of greater than 10, preferably greater than 12, and relatively hydrophobic surfactants e.g. having a HLB value of less than 10, preferably less than 8. Relatively hydrophilic surfactants include alkoxylate surfactants with an average in the range from about 10 to about 100 alkylene oxide, particularly ethylene oxide residues; and relatively hydrophobic surfactants include alkoxylate surfactants preferably with an average in the range from about 3 to about 10 alkylene oxide, particularly ethylene oxide residues.

Personal care formulations according to the invention can be divided by viscosity into milks and lotions, which preferably have a low shear viscosity (measured at shear rates of about 0.1 to 10 s$^{-1}$ as is typically used in Brookfield viscometers) of up to 10,000 mPa.s, and creams which preferably have a low shear viscosity of more than 10,000 mPa.s. Milks and lotions preferably have a low shear viscosity in the range from 100 to 10,000, more preferably 200 to 5,000, and particularly 300 to 1,000 mPa.s. The amount of composition according to the present invention present in a milk or lotion is preferably in the range from 2 to 3.5% by weight of the total formulation.

Creams preferably have a low shear viscosity of at least 20,000, more preferably in the range from 30,000 to 80,000, and particularly 40,000 to 70,000 mPa.s, although even higher viscosities e.g. up to about 10$^6$ mPa.s, may also be used. The amount of composition according to the present invention in a cream is preferably in the range from 2 to 3.5% by weight of the total formulation.

The emulsions of the invention may be made by generally conventional emulsification and mixing methods. For example, the composition of the invention may be added to (i) the oil phase, after which the aqueous phase is then added to the oil phase, or (ii) both the combined oil and water phases, or (iii) the water phase, which is then added to the oil phase. Method (i) is preferred. In all of these methods, the resulting mixture can then be emulsified using standard techniques. It is preferred to either heat the aqueous and oil phases usually above about 60ºC, e.g. to about 80 to 85ºC, or to subject the aqueous phase to high intensity mixing at lower, e.g. about ambient, temperature (cold process). Vigorous mixing and the use of moderately elevated temperatures can be combined if desired. The heating and/or high intensity mixing can be carried out before, during or after addition of the water to the oil phase.

The emulsions can also be made by inverse emulsification methods, whereby the composition of the invention is added to either the oil phase or the aqueous phase, and the aqueous phase is mixed into the oil phase to initially form a water in oil emulsion. Aqueous phase addition is continued until the system inverts to form an oil in water emulsion. Plainly a substantial amount of aqueous phase will generally be needed to effect inversion and so this method is not likely to be used for high oil phase content emulsions. Vigorous mixing and the use of moderately elevated temperatures can be combined if desired. Heating can be carried out during or after addition of the aqueous phase and before, during or after inversion. High intensity mixing can be carried out during or after addition of the aqueous phase, and before or during inversion

The emulsions may for example be microemulsions or nanoemulsions, having a mean droplet size over a wide range, preferably in the range from 10 to 10,000 nm. In one embodiment, the emulsion droplet size may be reduced, for example by high pressure homogenisation, preferably to a value in the range from 100 to 1,000 nm, more preferably 300 to 600 nm.

The emulsions according to the present invention are stable, measured as described herein, preferably for longer than one month, more preferably longer than two months, particularly longer than three months, and especially longer than four months at ambient temperature, and also preferably at 40 °C. The stability at even higher temperatures can be particularly important, and therefore the emulsion is stable, measured as described herein, suitably for longer than one week, preferably longer than two weeks, more preferably longer than 3 weeks, particularly longer than one month, and especially longer than two months at 50 °C, and also preferably at 60°C.

Personal Care formulations

The composition of the invention is preferably for use in a personal care formulation, more preferably a skin care product for example a sunscreen, cosmetic, antiperspirant, depilatory or dermatological product, or a hair care product for example a shampoo, conditioner, hair dye or hair relaxer product. In particular, the personal care formulation may be a formulation containing a high concentration of salts such as an antiperspirant deodorant, or a formulation having a low or high pH such as a depilatory or hair relaxer.

Many other components may be included in the formulation to make a personal care or cosmetic formulation or product. These components can be oil soluble, water soluble or non-soluble. Examples of such materials include:

(i) preservatives such as those based on potassium sorbate, sodium benzoate, parabens (alkyl esters of 4-hydroxybenzoic acid), phenoxyethanol, substituted ureas and hydantoin derivatives e.g. those sold commercially under the trade names Germaben II Nipaguard BPX and Nipaguard DMDMH. Such preservatives are used preferably at a concentration in the range from 0.5 to 2% by weight of the total composition. A preservative booster such as caprylyl glycol may also be used;

(ii) perfumes, when used preferably at a concentration in the range from 0.1 to 10% more preferably up to about 5%, and particularly up to about 2% by weight of the total composition;

(iii) humectants or solvents such as alcohols, polyols such as glycerol and polyethylene glycols, when used preferably at a concentration in the range from 1 to 10% by weight of the total composition;

(iv) sunfilter or sunscreen materials including organic sunscreens and/or inorganic sunscreens including those based on titanium dioxide or zinc oxide; when used preferably at a concentration in the range from 0.1% to 20%, more preferably 1 to 15%, and particularly 2 to 10% by weight of the total composition;

(v) alpha hydroxy acids such as glycolic, citric, lactic, malic, tartaric acids and their esters; self-tanning agents such as dihydroxyacetone;

(vi) antimicrobial, particularly anti-acne components such as salicylic acid;

(vii) vitamins and their precursors including: (a) Vitamin A, e.g. as retinyl palmitate and other tretinoin precursor molecules, (b) Vitamin B, e.g. as panthenol and its derivatives, (c) Vitamin C, e.g. as ascorbic acid and its derivatives, (d) Vitamin E, e.g. as tocopheryl acetate, (e) Vitamin F, e.g. as polyunsaturated fatty acid esters such as gamma-linolenic acid esters;

(viii) skin care agents such as ceramides either as natural materials or functional mimics of natural ceramides;

(ix) phospholipids, such as synthetic phospholipids or natural phospholipids, eg lecithin;

(x) vesicle-containing formulations;

(xi) germanium-containing compounds;

(xii) botanical extracts with beneficial skin care properties;

(xiii) skin whiteners such as Arlatone Dioic DCA (trade mark) sold by Croda, kojic acid, arbutin and similar materials;

(xiv) skin repair compounds actives such as Allantoin and similar series;

(xv) caffeine and similar compounds;

(xvi) cooling additives such as menthol or camphor;

(xvii) insect repellents such as N,N-diethyl-3-methylbenzamide (DEET) and citrus or eucalyptus oils;

(xviii) essential oils;

(xix) ethanol;

(xx) pigments, including microfine pigments, particularly oxides and silicates, e.g. iron oxide, particularly coated iron oxides, and/or titanium dioxide, and ceramic materials such as boron nitride;

(xxi) other solid components, such as are used in make up and cosmetics, to give suspoemulsions, preferably used in an amount in the range from 1 to 15 wt%, more preferably from 5 to 15 wt% based on the total weight of the formulation; and

(xxii) deodorant or antiperspirant agents, for example aluminium salts such as aluminium chlorohydrate. Such agents are typically present in a formulation at a concentration of up to 40% by weight (solids) based on the total weight of the formulation, preferably in the range from 1 to 40 wt% (solids), more preferably from 10 to 25 wt% (solids) based on the total weight of the formulation;

(xxiii) depilatory agents such as potassium thioglycollate or calcium thioglycollate. Such agents are typically present in a formulation at a concentration of up to 15% by weight (active) based on the total weight of the formulation, preferably between in the range from 1 to 15 wt% (active), more preferably from 2 to 7 wt% (active) based on the total weight of the formulation;

(xxiv) hair relaxing agents such as potassium hydroxide or sodium hydroxide. Such agents are typically present in a formulation at a concentration of up to 15% by weight based on the total weight of the formulation, preferably between in the range from 0.1 to 10 wt%, more preferably from 0.5 to 5 wt% (solids) based on the total weight of the formulation.

The composition and emulsions according to the present invention are suitable for use in a wide range of formulations and end-use applications, such as moisturizers, sunscreens, after sun products, body butters, gel creams, high perfume containing products, perfume creams, baby care products, hair treatments, hair conditioners, skin toning and skin whitening products, water-free products, anti-perspirant and deodorant products, tanning products, cleansers, 2-in-1 foaming emulsions, multiple emulsions, preservative free products, mild formulations, scrub formulations e.g. containing solid beads, silicone in water formulations, pigment containing products, sprayable emulsions, cosmetics, colour cosmetics, conditioners, shower products, foaming emulsions, make-up remover, eye make-up remover, and wipes.

The formulation may be a spray, lotion, cream or ointment. When the formulation is a colour cosmetic, it may be a foundation, mascara, eyeshadow or lipstick. The formulation may be an anti-perspirant or deodorant.

Formulations containing a composition or emulsion according to the present invention may have a pH value over a wide range, preferably in the range from 2 to 14, more preferably 2 to 8 or 6 to 13, and especially 2 to 5 or 8 to 13.

**Examples**

The invention is illustrated by the following non-limiting examples. All parts and percentages are given by weight unless otherwise stated.

It will be understood that all tests and physical properties listed have been determined at atmospheric pressure and ambient temperature (i.e. about 23°C), unless otherwise stated herein, or unless otherwise stated in the referenced test methods and procedures.

<u>Test Methods</u>

In this specification the following test methods have been used:

(i) Emulsion stability was assessed by observing the emulsions after storage for 3 months at ambient temperature (23°C), cold at 5°C or under elevated temperature storage at 40°C, 45°C and 50°C. Measuring storage stability at 50°C is a severe test. The emulsions were also assessed for their freeze-thaw stability using a cycling oven (-10°C to 40°C in 24 hours). The composition was stable if no visible separation of the emulsion occurred. The stability of the emulsions was also assessed by monitoring the size of the dispersed phase water particles over a three month period. The particle size was measured using a Malvern Mastersizer 2000 that measures the size of the dispersed phase particles using laser diffraction.

(ii) Emulsion viscosity was measured at 23ᵒC with a Brookfield LVT viscometer using an appropriate spindle (LV1, LV2, LV3, or LV4) depending on the viscosity of the emulsion. The emulsion was tested at 10 rpm (0.1 Hz), 1 day after making the emulsion and results are quoted in mPa.s.

(iii) The hydroxyl value is defined as the number of mg of potassium hydroxide equivalent to the hydroxyl content of 1 g of sample, and was measured by acetylation followed by hydrolysation of excess acetic anhydride. The acetic acid formed was subsequently titrated with an ethanolic potassium hydroxide solution.

(iv) The acid value is defined as the number of mg of potassium hydroxide required to neutralise the free acids in 1 g of sample, and was measured by direct titration with a standard potassium hydroxide solution.

(v) The iodine value is defined as the weight of iodine, $I_2$, in grams consumed by unsaturation in 100 g of sample. This is measured by reacting the sample with an excess of Wij's (Iodine monochloride) solution. The remaining Wij's solution is converted to Iodine with potassium iodide, the iodine is then titrated against a standard sodium thiosulphate solution.

(vi) Weight average molecular weight was determined by Gel Permeation Chromatography (GPC). The apparatus and settings used for the GPC are given in Table 1 below.

**Table 1:** GPC apparatus and settings

| Spectrometer | Polymer labs GPC-50 |
|---|---|
| Detector | Refractive index |
| Columns | PL gel 3$\mu$m 100A & PL gel 5$\mu$m mixed D |
| Solvent | Tetrahydrofuran (GPC grade). |
| Concentration of test substance | 1% |
| Colum temperature | 40 °C |
| Flow rate | 1ml per minute |
| Injection Volume | 100 micro litre |
| Analysis time | 25 minutes |
| Method Type Calibration | Area Normalisation<br>Relative, narrow standard calibration using PEG standards and a linear fit. The PEG standards had peak molecular weight (Mp) 106 to 3870 and were taken from an Aglient GPC/SEC Calibration kit, part number PL2070-0100 |

**Synthesis Examples**

The following synthesis methods are for making behenyl alcohol alkoxylates. The methods utilise a clean dry 10 litre pressure vessel equipped with agitation, a nitrogen sparge, thermometer, pressure gauge, and vacuum capability.

**Example 1** - **Behenyl alcohol + 1.2 moles EO + 0.3 moles PO**

Molten behenyl alcohol (1500 g, 4.65 mol) and 45% potassium hydroxide (11 g, 0.09 mol dry) were charged to the vessel, which was then purged with nitrogen and heated to 90°C with stirring. Vacuum was applied for one hour to dry the batch. The pressure was adjusted with nitrogen and ethylene oxide (304 g, 6.9 mol) was charged and reacted at 150°C for 2 hours. The batch was cooled to 120°C and propylene oxide (83 g, 1.4 mol) was charged and reacted at 120°C for 2 hours. The batch was then nitrogen stripped at 110°C for 1.5 hours, before cooling to 80°C, neutralising with lactic acid (~2 g) to a pH of 5.5-7.5 and discharging. The resultant product had hydroxyl value 145 mgKOH/g, with 1H NMR indicating a composition of behenyl alcohol + 1.2 moles EO + 0.3 moles PO.

**Example 2** - **Behenyl alcohol + 1.4 moles EO + 0.2 moles PO**

Molten behenyl alcohol (1500 g, 4.65 mol) and 45% potassium hydroxide (11 g, 0.09 mol dry) were charged to the vessel, which was then purged with nitrogen and heated to 90°C with stirring. Vacuum was applied for one hour to dry the batch. The pressure was adjusted with nitrogen and ethylene oxide (304 g, 6.9 mol) was charged and reacted at 150°C for 2 hours. The batch was cooled to 120°C and propylene oxide (83 g, 1.4 mol) was charged and reacted at 120°C for 2 hours. The batch was then nitrogen stripped at 110°C for 1.5 hours, before cooling to 80°C, neutralising with lactic acid (~2 g) to a pH of 5.5-7.5 and discharging. The resultant product had hydroxyl value 143 mgKOH/g, with 1H NMR indicating a composition of behenyl alcohol + 1.4 moles EO + 0.2 moles PO.

**Example 3 - Behenyl alcohol + 1.3 moles EO + 0.45 moles PO**

Molten behenyl alcohol (985 g, 3.05 mol) and 45% potassium hydroxide (5 g, 0.04 mol dry) were charged to the vessel, which was then purged with nitrogen and heated to 90°C with stirring. Vacuum was applied for one hour to dry the batch. The pressure was adjusted with nitrogen and ethylene oxide (180 g, 4.1 mol) was charged and reacted at 150°C for 2 hours. The batch was cooled to 120°C and propylene oxide (90 g, 1.55 mol) was charged and reacted at 120°C for 2 hours. The batch was then nitrogen stripped at 110°C for 1.5 hours, before cooling to 80°C, neutralising with lactic acid (~2 g) to a pH of 5.5-7.5 and discharging. The resultant product had hydroxyl value 140 mgKOH/g, with 1H NMR indicating a composition of behenyl alcohol + 1.3 moles EO + 0.45 moles PO.

**Example 4 - Behenyl alcohol + 3.6 moles EO + 0.3 moles PO**

Molten behenyl alcohol (1250 g, 3.9 mol) and 45% potassium hydroxide (9 g, 0.072 mol dry) were charged to the vessel, which was then purged with nitrogen and heated to 90°C with stirring. Vacuum was applied for one hour to dry the batch. The pressure was adjusted with nitrogen and ethylene oxide (710 g, 16.1 mol) was charged and reacted at 150°C for 2 hours. The batch was cooled to 120°C and propylene oxide (69 g, 1.4 mol) was charged and reacted at 120°C for 2 hours. The batch was then nitrogen stripped at 110°C for 1.5 hours, before cooling to 80°C, neutralising with lactic acid (~4 g) to a pH of 5.5-7.5 and discharging. The resultant product had hydroxyl value 114 mgKOH/g, with 1H NMR indicating a composition of behenyl alcohol + 3.6 moles EO + 0.3 moles PO.

**Example 5 - Behenyl alcohol + 26.8 moles EO**

Molten behenyl alcohol (1000 g, 3.1 mol) and 45% potassium hydroxide (11 g, 0.09 mol dry) were charged to the vessel, which was then purged with nitrogen and heated to 90°C with stirring. Vacuum was applied for one hour to dry the batch. The pressure was adjusted with nitrogen and ethylene oxide (4100 g, 93 mol) was charged and reacted at 150°C for 2 hours. The batch was then cooled to 80°C, neutralised with lactic acid (~2 g) to a pH of 5.5-7.5 and discharged. The resultant product had hydroxyl value 37.4 mgKOH/g, corresponding to behenyl alcohol + 26.8 moles EO.

**Comparative example A - Behenyl alcohol + 1.8 moles EO**

Molten behenyl alcohol (3200 g, 9.9 mol) and 45% potassium hydroxide (11 g, 0.09 mol dry) were charged to the vessel, which was then purged with nitrogen and heated to 90°C with stirring. Vacuum was applied for one hour to dry the batch. The pressure was adjusted with nitrogen and ethylene oxide (900 g, 20.5 mol) was charged and reacted at 150°C for 2 hours. The batch was then cooled to 80°C, neutralised with lactic acid (~3 g) to a pH of 5.5-7.5 and discharged. The resultant product had hydroxyl value 138.4 mgKOH/g, corresponding to behenyl alcohol + 1.8 moles EO.

**Comparative example B - Behenyl alcohol + 4.5 moles EO**

Molten behenyl alcohol (1970 g, 6.1 mol) and 45% potassium hydroxide (11 g, 0.09 mol dry) were charged to the vessel, which was then purged with nitrogen and heated to 90°C with stirring. Vacuum was applied for one hour to dry the batch. The pressure was adjusted with nitrogen and ethylene oxide (1350 g, 30.7 mol) was charged and reacted at 150°C for 2 hours. The batch was then cooled to 80°C, neutralised with lactic acid (~4 g) to a pH of 5.5-7.5 and discharged. The resultant product had hydroxyl value 107.8 mgKOH/g, corresponding to behenyl alcohol + 4.5 moles EO.

**Example 6 - Further alcohol alkoxylates**

Further alcohol alkoxylates were prepared in the same way as described in Examples 1 to 5 and the products are given in Table 2 below.

Table 2 - further alcohol alkoxylate Examples

| Fatty alcohol | Moles EO | Moles PO | Hydroxyl value |
|---|---|---|---|
| 1-Octadecanol (C18) | 1.3 | 0.3 | 162.6 |
| 1 -Octadecanol (C18) | 3.5 | 0.3 | 127.0 |
| 1-Octadecanol (C18) | 29.4 | 0 | 35.9 |
| 1-Eicosanol (C20) | 1.4 | 0.3 | 146.1 |
| 1-Eicosanol (C20) | 3.8 | 0.3 | 114.6 |
| 1-Eicosanol (C20) | 28.6 | 0 | 35.8 |
| 1-Tetracosanol (C24) | 1.2 | 0.2 | 133.7 |
| 1-Tetracosanol (C24) | 3.7 | 0.3 | 104.8 |
| 1-Tetracosanol (C24) | 29.1 | 0 | 34.3 |

**Emulsifier System Examples**

**Examples 7-9 - Emulsifier systems**

The following emulsifier systems / compositions were produced by blending the compounds of Examples 1 to 5 in the proportions defined below.

| Emusifier system | Compound of Example 1 (w/w%) | Compound of Example 2 (w/w%) | Compound of Example 3 (w/w%) | Compound of Example 4 (w/w%) | Compound of Example 5 (w/w%) |
|---|---|---|---|---|---|
| Example 7 | 64 | - | - | 16 | 20 |
| Example 8 | - | 64 | - | 16 | 20 |
| Example 9 | - | - | 64 | 16 | 20 |

**Formulation Examples**

**Example 10 - Anti-Acne Cream**

| Product / INCI Name | Functionality | % w/w |
|---|---|---|
| **Phase A** | | |
| **Arlamol PS15E** (PPG-15 Stearyl Ether) | Emollient | 8.00 |
| **Pristerene 9559** (Stearic acid) | Moisturising agent | 2.00 |

(continued)

| Product / INCI Name | Functionality | % w/w |
|---|---|---|
| **Phase A** | | |
| **Crodacol CS90 EP** (Cetearyl alcohol) | Emulsion stabiliser | 1.50 |
| Xiameter PMX-200 - 20 cst (Dimethicone) | Emollient | 0.30 |
| Emulsifier system of example 7 | Emulsifier | 3.00 |
| **Phase B** | | |
| Deionised water | | to 100 % |
| **Renex S30** (Sorbeth-30) | Humectant | 4.00 |
| Structure Zea (Hydroxypropyl Starch Phosphate) | Thickener | 3.50 |
| **Arlasilk EFA TM** (Linoleamidopropyl PG-Dimonium Chloride Phosphate) | Phophate emulsifier | 3.00 |
| **Phase C** | | |
| **Arlasolve DMI TM** (Dimethyl Isosorbide) | Solvent | 5.00 |
| Salicylic acid | Anti-acne active | 1.00 |
| **Phase D** | | |
| Preservative | Preservative | qs |

The Structure Zea was dispersed in water. Phases A and B were separately mixed and heated to 75°C. Phase A was then added to phase B slowly with moderate stirring. The resulting mixture was homogenised for 1 minute at 9,500 rpm before being allowed to cool to 40°C with moderate stirring. Phase C and then phase D were added while continuing to stir gently until the emulsion reached room temperature.

**Example 11 - High Oil Content Cream**

| Product / INCI Name | Functionality | % w/w |
|---|---|---|
| **Phase A** | | |
| Paraffin Oil Perliquidum (Mineral oil) | Occlusive oil | 70.0 |
| Emulsifier system of example 8 | Emulsifier | 3.00 |
| **Phase B** | | |
| Deionised water | | to 100% |
| **Phase C** | | |
| Germaben II (Propylene Glycol, Diazolidinyl Urea, Methylparaben, Propylparaben) | Preservative | 1.00 |
| **Phase D** | | |
| Carbomer (2% aqueous solution) | Thickener | 5.00 |
| **Phase E** | | |
| Sodium Hydroxide (10% aq) (Water (and ) Sodium Hydroxide) | pH adjuster | qs |

Phases A and B were separately mixed and heated to 70°C. Phase C was added to phase B just prior to emulsification. Slowly phase A was added to phase B whilst stirring intensively, and then homogenised for 1 minute. The emulsion was allowed to cool to 50°C with moderate stirring, at which point phase D was added whilst stirring intensively. The formulation was neutralised with phase Eand allowed to cool to room temperature with moderate stirring.

**Example 12 - Deodorant**

| Product (INCI Name) | Functionality | % w/w |
|---|---|---|
| **Phase A** | | |
| **Arlamol™ PS15E** (PPG-15 Stearyl Ether)[1] | Emollient | 3.00 |
| Emulsifier system of example 9 | O/W Emulsifier | 3.00 |

| | | |
|---|---|---|
| **Crodacol™ CS90** (Cetearyl Alcohol)[1] | Emulsion stabiliser, viscosity builder | 0.65 |
| **Super Sterol Liquid™** (C10-30 Cholesterol/Lanosterol Esters)[1] | Skin repair and moisturising agent | 0.50 |
| **Phase B** | | |
| Water Deionised (Aqua) | - | To 100 |
| **Arlasilk™ PTM** (Myristamidopropyl PG-Dimonium Chloride Phosphate (and) Aqua)[1] | Preservative Booster | 1.00 |
| Reach 301 Aluminium Chlorohydrate Solution (Aluminium Sesquichlorohydrate)[3] | Antiperspirant active | 32.00 |
| Euxyl PE9010 (Phenoxyethanol (and) Ethylhexylglycerin)[4] | Preservative | 0.80 |

The Arlasilk PTM was added to the water before beginning to heat to 70-75°C. Separately the oil phase was combined and heated to 70-75°C. The oil phase was added to the water and Arlasilk PTM mixture with high speed stirring, and then homogenised for one minute. The stirring was slowed whilst the mixture cooled and the remaining ingredients added at <40°C.

**Example 13** - **Fake Tan Lotion**

| Product | Function | %w/w |
|---|---|---|
| **Phase A** | | |
| Polawax NF | Nonionic Emulsifying Wax | 5.0 |
| Crodamol PMP (PPG-2 Myristyl Ether Propionate) | Emollient | 3.0 |
| Mineral oil | Emollient | 3.0 |
| Emulsifier system of example 7 | Emulsifier | 3.00 |
| Silicone Fluid DC344 | Silicon | 1.0 |
| **Phase B** | | |
| Water Deionised (Aqua) | | To 100 |
| Propylene Glycol | Humectant | 1.0 |
| | | |
| **Phase C** | | |
| Water | | 7.0 |
| Dihydroxyacetone | Active | 5.0 |

14

The Dihydroxyacetone was pre-blended in the water of phase C. Phase A and phase B were separately mixed and heated to 75-80°C. Phases A and B were then combined with stirring and allowed to cool. Phase C and perfume was added into the mixture of phases A and B at 35-40°C. The formulation had a final pH of 4-6.

**Example 14 - Hair Conditioner**

| Product / INCI Name | Functionality | % w/w |
|---|---|---|
| **Phase A** | | |
| **ChromAveil™** (Quaternium-95 (and) Propanediol)[1] | UV absorber | 3.00 |
| **Crodasorb™ UV-HPP** (Polyquaternium-59 (and) Butylene Glycol)[1] | UV-B absorber | 3.00 |
| **Crodacol™ C90** (Cetyl Alcohol)[1] | Viscosity builder | 2.50 |
| **KeraDyn™ HH** (Bis-Ethyl(isostearylimidazoline) Isostearamide)[1] | Conditioning agent | 2.00 |
| **Crodamol™ GTIS** (Triisostearin)[1] | Emollient | 2.00 |
| **Crodamol™ SS** (Cetyl Esters Wax)[1] | Emollient | 2.00 |
| **Crodacol™ CS90** (Cetearyl Alcohol)[1] | Viscosity builder | 2.00 |
| Emulsifier system of example 8 | o/w emulsifier | 3.00 |
| **Croodamol™ STS** (PPG 3 Benzyl Ether Myristate)[1] | Emollient | 1.50 |
| **Phase B** | | |
| Water Deionised (Aqua) | - | To 100 |
| Preservative | - | qs |
| Fragrance | - | qs |
| Lactic acid | pH adjuster | To pH 4-4.5 |

Separately phases A and B were combined. Both phases were heated to 65-70 °C and then mixed while stirring at 500rpm. The stirring was then reduced to 300rpm whilst the mixture cooled. Once cool the preservative, fragrance and lactic acid were added as required.

**Example 15 - Roll-on antiperspirant**

| Product | %w/w |
|---|---|
| **Phase A** | |
| Emulsifier system of example 9 | 3.00 |
| CRODAMOL PMP | 2.0 |
| CRODACOL CS90 EP | 1.0 |
| **Phase B** | |
| Water Deionised (Aqua) | To 100 |
| Chlorhydrol 50% Solution (Aluminium Chlorohydrate) | 38.0 |
| Magnesium Aluminium Silicate | 1.0 |
| Propylene glycol | 1.0 |

The magnesium aluminium silicate was hydrated in warm water. The oil phase and the rest of the water phase

15

were then mixed separately and heated to 70-75°C. The dispersed magnesium aluminium silicate phase was added to the warm water phase, and then the oil phase was added to the water phase under stirring. Perfume was added to the formulation once it had cooled to 40°C, and the completed formulation allowed to cool while stirring.

**Example 16 - Depilatory Lotion**

| Product | % w/w |
|---|---|
| **Phase A** | |
| Crodacol CS90 EP (Cetearyl Alcohol) | 5.0 |
| Emulsifier system of example 7 | 3.00 |
| Mineral oil | 1.5 |
| **Phase B** | |
| Water Deionised (Aqua) | To 100 |
| Potassium Thioglycollate (30% Aqueous Solution) | 15.0 |
| Hydroxyethyl Cellulose | 0.2 |
| **Phase C** | |
| Water Deionised (Aqua) | 14.0 |
| Calcium Hydroxide | 0.5 |
| Potassium Hydroxide (30% Aqueous Solution) | To pH 12.5 |

The hydroxyethyl cellulose was hydrated in warm water (60-65°C). The oil phase was mixed and heated to 60-65°C, then the cellulose solution was added to the oil phase whilst stirring. The mixture was stirred to cool, adding potassium thioglycollate solution once the temperature of the mixture reached 30-35°C. The calcium hydroxide was slurried into the remaining water and added to the emulsion. The pH of the emulsion was adjusted. Finally, the emulsion was stirred and cooled.

**Example 17 - Potassium Hydroxide Hair Relaxer**

| Product | %w/w |
|---|---|
| **Phase A** | |
| Mineral Oil 25cS at 25°C (Paraffinum Liquidum) | 15.0 |
| Emulsifier system of example 9 | 3.00 |
| White Petroleum Jelly | 4.0 |
| Crodacol C90 | 1.0 |
| **Phase B** | |
| Water | To 100 |
| Propylene glycol | 2.0 |
| **Phase C** | |
| Water | 15.0 |
| Potassium hydroxide | 2.4 |

The oil phase and water phase were heated separately to 65-70 °C. The water phase was then added to the oil phase with stirring. The mixture was allowed to cool while being stirred. At 35°C the Potassium Hydroxide solution was added and the formulation filled off at 30°C.

**Example 18 - Lye Relaxer Cream**

| Product / INCI Name | Functionality | % w/w |
|---|---|---|
| OIL PHASE | | |
| Emulsifier system of example 8 | Emulsifier | 3.00 |
| Mineral Oil (Paraffinum Liquidum) | Oil | 18 |
| Petrolatum Jelly (Petroleum) | Fat | 13 |
| Procetyl AWS (PPG-5 Ceteth-20) | Emulsifier | 1.8 |
| Crodacol S95 (Stearyl Alcohol) | Consistency agent | 2 |
| Crodacol C90 (Cetyl Alcohol) | Consistency agent | 1 |
| Crodamol STS (PPG-3 Benzyl Ether Myristate) | Shine effect | 2 |
| Propyl Paraben | Preservative | 0.15 |
| WATER PHASE | | |
| Water | Solvent | To 100.0 |
| Propylene Glycol | Preservative | 3 |
| Solan E (PEG-75 Lanolin) | Protective agent | 0.5 |
| Methyl Paraben | Preservative | 0.15 |
| NaOH solution - 21.7% | Relaxing agent | 10 |
| Menthol | Cooling agent | 0.15 |
| Croquat L (Lauryldimonium Hydroxypropyl Hydrolyzed Collagen) | Protective agent | 0.5 |

The oil phase and water phase were mixed and heated separately to 70-75°C. The water phase was added to the oil phase with gentle stirring. Once below 50°C, the remaining ingredients were added under stirring.

**Example 19 - Hair Relaxer**

| Ingredient/INCI Name | Functionality | % w/w |
|---|---|---|
| Part A | | |
| Emulsifier system of example 7 | | 3.00 |
| Petroleum Jelly (Petrolatum)[2] | Occlusive | 18 |
| Mineral Oil (Paraffinum Liquidum)[2] | Occlusive | 13 |
| Part B | | |
| Water Deionised (Aqua) | - | 48.9 |
| Propylene Glycol[2] | Humectant | 2 |
| Part C | | |
| Water Deionised (Aqua) | - | 6 |
| Sodium Hydroxide[3] | Hair Relaxing Active | 2.1 |

17

Part A and Part B were separately mixed and heated to 65°C. Part B was added to Part A with mixing and cooled to 40°C. The ingredients of Part C were combined with mixing, and cooled to room temperature. The Part A/B mixer was switched to a side sweep blade. Slowly Part C was added to Part A/B, continuing mixing for 30 minutes until completely smooth and homogenous. The formulation was then cooled further to the desired fill temperature.

**Example 20 - Sodium Hydroxide Relaxer**

| Product (INCI Name) | Functionality | % w/w |
|---|---|---|
| **Part A** | | |
| Emulsifier system of example 8 | | 3.00 |
| Mineral OIl (Paraffinum Liquidum) | Occlusive | 10 |
| White Petroleum Jelly (Petrolatum) | Occlusive | 12 |
| **Crodacol CS90**[1](Cetearyl Alcohol) | Non-ionic co-emulsifer and viscosity builder | 1 |
| **Part B** | | |
| Water Deionised (Aqua) | | to 100 |
| Propylene Glycol | Humectant | 2 |
| **Solan 75**[1] (PEG-75 Lanolin) | Superfatting agent | 3 |
| **Part C** | | |
| Water Deionised (Aqua) | | 10 |
| Sodium Hydroxyde | Hair relaxing active | 1.95 |

The oil phase and water phase were mixed and heated separately to 60-65°C. The water phase was added to the oil phase with stirring. In a separate container, the Sodium Hydroxide was premixed with the water quantity. The emulsion was stirred to cool, and the Sodium Hydroxide solution added in at 40-45°C. Stirring was continued whilst cooling to the desired fill off temperature.

**Example 21 - Low pH Hair Straightening Emulsion**

| Product | %w/w |
|---|---|
| **Part A** | |
| Cetearyl Alcohol | 2.00 |
| Emulsifier system of example 9 | *1.00 - 3.00* |
| Polyquaternium 7 | 0.50 |
| MethylChlorolsothiazolinone/Methylisotiazolinone | 0.10 |
| Mineral oil | 2.50 |
| Dimethicone | 1.00 |
| **Part B** | |
| Water | To 100 |
| Glycerin | 5.00 |
| **Part C** | |
| Glyoxylic Acid | 15.00 |

Separately parts A and B were combined and heated to - 70°C. Part A was then added to part B with stirring. The emulsion was then allowed to cool, and part C added once the temperature fell below 45°C.

**Claims**

1.   A composition comprising:

a) an alkoxylated fatty alcohol compound of formula (A)

$$R^1(OC_2H_4)_n(OC_3H_6)_m \qquad (A)$$

wherein $R^1$ is a $C_{16}$-$C_{26}$ alkyl or alkenyl group,
n is the mean number of moles of -$(OC_2H_4)$- per molecule of formula (A) present in the compound and is between 15 and 60,
m is the mean number of moles of -$(OC_3H_6)$- per molecule of formula (A) present in the compound and is between 0 and 20,
and n+m > 15 ; and

b) an alkoxylated fatty alcohol compound of formula (B)

$$R^2(OC_2H_4)_r(OC_3H_6)_s \qquad (B)$$

wherein $R^2$ is a $C_{16}$-$C_{26}$ alkyl or alkenyl group,
r is the mean number of moles of -$(OC_2H_4)$- per molecule of formula (B) present in the compound and is between 0.01 and 14.99,
s is the mean number of moles of -$(OC_3H_6)$- per molecule of formula (B) present in the compound and is between 0.01 and 14.99, and

$$r+s \leq 15.$$

2.   The composition according to claim 1, wherein the $R^1$ and $R^2$ are residues of a fatty alcohol or mixture of fatty alcohols, said fatty alcohols independently selected from cetyl alcohol, cetostearyl alcohol, palmitoleyl alcohol, stearyl alcohol, isostearyl alcohol, oleyl alcohol, arachidyl alcohol, behenyl alcohol, isobehenyl alcohol, erucyl alcohol, lignceryl alcohol and cerotinyl alcohol.

3.   The composition according to either claim 1 or claim 2, wherein n is between 15 and 60 and m is between 0 and 10.

4.   The composition according to any preceding claim, wherein r is between 0.1 and 10 and s is between 0.05 and 10.

5.   The composition according to any preceding claim, wherein the compound of formula (B) may be a mixture of components (B') and (B") having the formulas:

$$R^2(OC_2H_4)_r \qquad (B')$$

and

$$R^2(OC_2H_4)_r(OC_3H_6)_t \qquad (B"),$$

wherein $R^2$ and r are defined in accordance with claim 1, and t is the mean number of moles of -$(OC_3H_6)$ per molecule of formula (B") present in the compound and is between 0.01 and 14.99.

6.   The composition according to any preceding claim, wherein the composition further comprises an alkoxylated fatty alcohol compound of formula (C)

$$R^3(OC_2H_4)_x(OC_3H_6)_y \qquad (C)$$

wherein $R^3$ is a $C_{16}$-$C_{26}$ alkyl or alkenyl group,

x is the mean number of moles of -$(OC_2H_4)$- per molecule of formula (C) present in the compound and is between 0.01 and 14.99,

y is the mean number of moles of -$(OC_3H_6)$- per molecule of formula (C) present in the compound and is between 0.01 and 14.99, and

$$x+y \leq 15 \neq r+s.$$

7. The composition according to any preceding claim, wherein the mean number of moles of component b) to component a) is in the range from 0.1 to 8:1.

8. The composition according to any preceding claim, wherein the composition comprises a maximum of 10% by weight of water.

9. The composition according to any preceding claim, wherein the composition has an acid value in the range from 0 to 15 mgKOH/g.

10. The composition according to any preceding claim, wherein the composition has a hydroxyl (OH) value in the range from 50 to 300 mgKOH/g.

11. An emulsifier system comprising a composition according to any of claims 1 to 10.

12. A personal care formulation comprising a composition according to any of claims 1 to 10 or an emulsifier system according to claim 11.

13. The formulation according to claim 12, wherein the amount of composition or emulsifier system is in the range from 0.1 to 10% by weight of the total formulation.

14. A method of stabilising an emulsion comprising the step of mixing an emulsifier system according to claim 11 or a composition with the emulsion, the composition comprising:

   a) an alkoxylated fatty alcohol compound of formula (A)

   $$R^1(OC_2H_4)_n(OC_3H_6)_m \qquad (A)$$

   wherein $R^1$ is a $C_{16}$-$C_{26}$ alkyl or alkenyl group,
   n is the mean number of moles of -$(OC_2H_4)$- per molecule of formula (A) present in the compound and is between 15 and 100,
   m is the mean number of moles of -$(OC_3H_6)$- per molecule of formula (A) present in the compound and is between 0 and 20,
   and n+m > 15 ; and

   b) an alkoxylated fatty alcohol compound of formula (B)

   $$R^2(OC_2H_4)_r(OC_3H_6)_s \qquad (B)$$

   wherein $R^2$ is a $C_{16}$-$C_{26}$ alkyl or alkenyl group,
   r is the mean number of moles of -$(OC_2H_4)$- per molecule of formula (B) present in the compound and is between 0.01 and 14.99,
   s is the mean number of moles of -$(OC_3H_6)$- per molecule of formula (B) present in the compound and is between 0.01 and 14.99, and

$$r+s \leq 15.$$

15. Use of an emulsifier system according to claim 11 or a composition to stabilise an emulsion, the composition comprising:

a) an alkoxylated fatty alcohol compound of formula (A)

$$R^1(OC_2H_4)_n(OC_3H_6)_m \qquad (A)$$

wherein $R^1$ is a $C_{16}$-$C_{26}$ alkyl or alkenyl group,
n is the mean number of moles of -$(OC_2H_4)$- per molecule of formula (A) present in the compound and is between 15 and 100,
m is the mean number of moles of -$(OC_3H_6)$- per molecule of formula (A) present in the compound and is between 0 and 20,
and n+m > 15 ; and

b) an alkoxylated fatty alcohol compound of formula (B)

$$R^2(OC_2H_4)_r(OC_3H_6)_s \qquad (B)$$

wherein $R^2$ is a $C_{16}$-$C_{26}$ alkyl or alkenyl group,
r is the mean number of moles of -$(OC_2H_4)$- per molecule of formula (B) present in the compound and is between 0.01 and 14.99,
s is the mean number of moles of -$(OC_3H_6)$- per molecule of formula (B) present in the compound and is between 0.01 and 14.99, and

$$r+s \leq 15.$$

**Patentansprüche**

1. Zusammensetzung, umfassend:

a) eine alkoxylierte Fettalkoholverbindung der Formel (A)

$$R^1(OC_2H_4)_n(OC_3H_6)_m \qquad (A),$$

wobei $R^1$ für eine $C_{16}$-$C_{26}$-Alkyl- oder -Alkenylgruppe steht,
n für die mittlere Zahl der Mole von -$(OC_2H_4)$- pro Molekül der Formel (A) in der Verbindung steht und zwischen 15 und 60 liegt,
m für die mittlere Zahl der Mole von -$(OC_3H_6)$- pro Molekül der Formel (A) in der Verbindung steht und zwischen 0 und 20 liegt
und n + m > 15; und

b) eine alkoxylierte Fettalkoholverbindung der Formel (B)

$$R^2(OC_2H_4)_r(OC_3H_6)_s \qquad (B),$$

wobei $R^2$ für eine $C_{16}$-$C_{26}$-Alkyl- oder -Alkenylgruppe steht,
r für die mittlere Zahl der Mole von -$(OC_2H_4)$- pro Molekül der Formel (B) in der Verbindung steht und zwischen 0,01 und 14,99 liegt,
s für die mittlere Zahl der Mole von -$(OC_3H_6)$- pro Molekül der Formel (B) in der Verbindung steht und zwischen 0,01 und 14,99 liegt und

$$r + s \leq 15.$$

2. Zusammensetzung nach Anspruch 1, wobei $R^1$ und $R^2$ für Reste eines Fettalkohols oder Gemischs von Fettalkoholen stehen, wobei die Fettalkohole unabhängig aus Cetylalkohol, Cetostearylalkohol, Palmitoleylalkohol, Stearylalkohol, Isostearylalkohol, Oleylalkohol, Arachidylalkohol, Behenylalkohol, Isobehenylalkohol, Erucylalkohol, Lignocerylalkohol und Cerotinylalkohol ausgewählt sind.

3. Zusammensetzung nach Anspruch 1 oder Anspruch 2, wobei n zwischen 15 und 60 liegt und m zwischen 0 und 10 liegt.

4. Zusammensetzung nach einem der vorhergehenden Ansprüche, wobei r zwischen 0,1 und 10 liegt und s zwischen 0,05 und 10 liegt.

5. Zusammensetzung nach einem der vorhergehenden Ansprüche, wobei es sich bei der Verbindung der Formel (B) um ein Gemisch von Komponenten (B') und (B") mit den Formeln:

$$R^2(OC_2H_4)_r \qquad (B')$$

und

$$R^2(OC_2H_4)_r(OC_3H_6)_t \qquad (B")$$

handelt, wobei $R^2$ und r gemäß Anspruch 1 definiert sind und t für die mittlere Zahl der Mole von $-(OC_3H_6)-$ pro Molekül der Formel (B") in der Verbindung steht und zwischen 0,01 und 14,99 liegt.

6. Zusammensetzung nach einem der vorhergehenden Ansprüche, wobei die Zusammensetzung ferner eine alkoxylierte Fettalkoholverbindung der Formel (C)

$$R^3(OC_2H_4)_x(OC_3H_6)_y \qquad (C)$$

umfasst, wobei $R^3$ für eine $C_{16}$-$C_{26}$-Alkyl- oder -Alkenylgruppe steht,
x für die mittlere Zahl der Mole von $-(OC_2H_4)-$ pro Molekül der Formel (C) in der Verbindung steht und zwischen 0,01 und 14,99 liegt,
y für die mittlere Zahl der Mole von $-(OC_3H_6)-$ pro Molekül der Formel (C) in der Verbindung steht und zwischen 0,01 und 14,99 liegt und

$$x + y \leq 15 \neq r + s.$$

7. Zusammensetzung nach einem der vorhergehenden Ansprüche, wobei die mittlere Zahl der Mole von Komponente b) zur Komponente a) im Bereich von 0,1 bis 8:1 liegt.

8. Zusammensetzung nach einem der vorhergehenden Ansprüche, wobei die Zusammensetzung maximal 10 Gew.-% Wasser umfasst.

9. Zusammensetzung nach einem der vorhergehenden Ansprüche, wobei die Zusammensetzung eine Säurezahl im Bereich von 0 bis 15 mg KOH/g aufweist.

10. Zusammensetzung nach einem der vorhergehenden Ansprüche, wobei die Zusammensetzung eine Hydroxylzahl (OH-Zahl) im Bereich von 50 bis 300 mg KOH/g aufweist.

11. Emulgatorsystem, umfassend eine Zusammensetzung nach einem der Ansprüche 1 bis 10.

12. Körperpflegeformulierung, umfassend eine Zusammensetzung nach einem der Ansprüche 1 bis 10 oder ein Emulgatorsystem nach Anspruch 11.

**13.** Formulierung nach Anspruch 12, wobei die Menge an Zusammensetzung oder Emulgatorsystem im Bereich von 0,1 bis 10 Gew.-% der gesamten Formulierung liegt.

**14.** Verfahren zum Stabilisieren einer Emulsion, umfassend den Schritt des Mischens eines Emulgatorsystems nach Anspruch 11 oder einer Zusammensetzung mit der Emulsion, wobei die Zusammensetzung Folgendes umfasst:

a) eine alkoxylierte Fettalkoholverbindung der Formel (A)

$$R^1(OC_2H_4)_n(OC_3H_6)_m \qquad (A),$$

wobei $R^1$ für eine $C_{16}$-$C_{26}$-Alkyl- oder -Alkenylgruppe steht,
n für die mittlere Zahl der Mole von -$(OC_2H_4)$- pro Molekül der Formel (A) in der Verbindung steht und zwischen 15 und 100 liegt,
m für die mittlere Zahl der Mole von -$(OC_3H_6)$- pro Molekül der Formel (A) in der Verbindung steht und zwischen 0 und 20 liegt
und n + m > 15; und

b) eine alkoxylierte Fettalkoholverbindung der Formel (B)

$$R^2(OC_2H_4)_r(OC_3H_6)_s \qquad (B),$$

wobei $R^2$ für eine $C_{16}$-$C_{26}$-Alkyl- oder -Alkenylgruppe steht,
r für die mittlere Zahl der Mole von -$(OC_2H_4)$- pro Molekül der Formel (B) in der Verbindung steht und zwischen 0,01 und 14,99 liegt,
s für die mittlere Zahl der Mole von -$(OC_3H_6)$- pro Molekül der Formel (B) in der Verbindung steht und zwischen 0,01 und 14,99 liegt und

$$r + s \leq 15.$$

**15.** Verwendung eines Emulgatorsystems nach Anspruch 11 oder einer Zusammensetzung zur Stabilisierung einer Emulsion, wobei die Zusammensetzung Folgendes umfasst:

a) eine alkoxylierte Fettalkoholverbindung der Formel (A)

$$R^1(OC_2H_4)_n(OC_3H_6)_m \qquad (A),$$

wobei $R^1$ für eine $C_{16}$-$C_{26}$-Alkyl- oder -Alkenylgruppe steht,
n für die mittlere Zahl der Mole von -$(OC_2H_4)$- pro Molekül der Formel (A) in der Verbindung steht und zwischen 15 und 100 liegt,
m für die mittlere Zahl der Mole von -$(OC_3H_6)$- pro Molekül der Formel (A) in der Verbindung steht und zwischen 0 und 20 liegt
und n + m > 15; und

b) eine alkoxylierte Fettalkoholverbindung der Formel (B)

$$R^2(OC_2H_4)_r(OC_3H_6)_s \qquad (B),$$

wobei $R^2$ für eine $C_{16}$-$C_{26}$-Alkyl- oder -Alkenylgruppe steht,
r für die mittlere Zahl der Mole von -$(OC_2H_4)$- pro Molekül der Formel (B) in der Verbindung steht und zwischen 0,01 und 14,99 liegt,
s für die mittlere Zahl der Mole von -$(OC_3H_6)$- pro Molekül der Formel (B) in der Verbindung steht und zwischen 0,01 und 14,99 liegt und

$$r + s \leq 15.$$

## Revendications

1. Composition comprenant :

   a) un composé de type alcool gras alcoxylé de formule (A)

   $$R^1(OC_2H_4)_n(OC_3H_6)_m \qquad (A)$$

   $R^1$ étant un groupe alkyle ou alcényle en $C_{16-26}$,
   n étant le nombre moyen de moles de $-(OC_2H_4)-$ par molécule de formule (A) présente dans le composé et étant compris entre 15 et 60,
   m étant le nombre moyen de moles de $-(OC_3H_6)-$ par molécule de formule (A) présente dans le composé et étant compris entre 0 et 20,
   et n + m > 15 ; et

   b) un composé de type alcool gras alcoxylé de formule (B)

   $$R^2(OC_2H_4)_r(OC_3H_6)_s \qquad (B)$$

   $R^2$ étant un groupe alkyle ou alcényle en $C_{16-26}$,
   r étant le nombre moyen de moles de $-(OC_2H_4)-$ par molécule de formule (B) présente dans le composé et étant compris entre 0,01 et 14,99,
   s étant le nombre moyen de moles de $-(OC_3H_6)-$ par molécule de formule (B) présente dans le composé et étant compris entre 0,01 et 14,99, et

   $$r + s \leq 15.$$

2. Composition selon la revendication 1, les $R^1$ et $R^2$ étant des résidus d'un alcool gras ou d'un mélange d'alcools gras, lesdits alcools gras étant indépendamment choisis parmi l'alcool cétylique, l'alcool cétostéarylique, l'alcool palmitoléylique, l'alcool stéarylique, l'alcool isostéarylique, l'alcool oléylique, l'alcool arachidylique, l'alcool béhénylique, l'alcool isobéhénylique, l'alcool érucylique, l'alcool lignocéryléique et l'alcool cérotinylique.

3. Composition selon l'une ou l'autre parmi la revendication 1 ou la revendication 2, n étant compris entre 15 et 60 et m étant compris entre 0 et 10.

4. Composition selon une quelconque revendication précédente, r étant compris entre 0,1 et 10 et s étant compris entre 0,05 et 10.

5. Composition selon une quelconque revendication précédente, le composé de formule (B) pouvant être un mélange de composants (B') et (B") ayant les formules :

   $$R^2(OC_2H_4)_r \qquad (B')$$

   et

   $$R^2(OC_2H_4)_r(OC_3H_6)_t \qquad (B")$$

   , $R^2$ et r étant définis conformément à la revendication 1, et t étant le nombre moyen de moles de $-(OC_3H_6)-$par molécule de formule (B") présente dans le composé et étant compris entre 0,01 et 14,99.

6. Composition selon une quelconque revendication précédente, la composition comprenant en outre un composé de type alcool gras alcoxylé de formule (C)

$$R^3(OC_2H_4)_x(OC_3H_6)_y \qquad (C)$$

$R^3$ étant un groupe alkyle ou alcényle en $C_{16-26}$,
x étant le nombre moyen de moles de $-(OC_2H_4)-$ par molécule de formule (C) présente dans le composé et étant compris entre 0,01 et 14,99,
y étant le nombre moyen de moles de $-(OC_3H_6)-$ par molécule de formule (C) présente dans le composé et étant compris entre 0,01 et 14,99, et

$$x + y \leq 15 \neq r + s.$$

**7.** Composition selon une quelconque revendication précédente, le nombre moyen de moles de composant b) sur composant a) étant dans la plage de 0,1 à 8 : 1.

**8.** Composition selon une quelconque revendication précédente, la composition comprenant un maximum de 10 % en poids d'eau.

**9.** Composition selon une quelconque revendication précédente, la composition ayant un indice d'acide dans la plage de 0 à 15 mg de KOH/g.

**10.** Composition selon une quelconque revendication précédente, la composition ayant un indice d'hydroxyle (OH) dans la plage de 50 à 300 mg de KOH/g.

**11.** Système d'émulsifiant comprenant une composition selon l'une quelconque des revendications 1 à 10.

**12.** Formulation de soin personnel comprenant une composition selon l'une quelconque des revendications 1 à 10 ou un système d'émulsifiant selon la revendication 11.

**13.** Formulation selon la revendication 12, la quantité de composition ou de système d'émulsifiant étant dans la plage de 0,1 à 10 % en poids de la formulation totale.

**14.** Procédé de stabilisation d'une émulsion comprenant l'étape de mélange d'un système d'émulsifiant selon la revendication 11 ou d'une composition avec l'émulsion, la composition comprenant :

a) un composé de type alcool gras alcoxylé de formule (A)

$$R^1(OC_2H_4)(OC_3H_6)_m \qquad (A)$$

$R^1$ étant un groupe alkyle ou alcényle en $C_{16-26}$,
n étant le nombre moyen de moles de $-(OC_2H_4)-$ par molécule de formule (A) présente dans le composé et étant compris entre 15 et 100,
m étant le nombre moyen de moles de $-(OC_3H_6)-$ par molécule de formule (A) présente dans le composé et étant compris entre 0 et 20,
et n + m > 15 ; et

b) un composé de type alcool gras alcoxylé de formule (B)

$$R^2(OC_2H_4)_r(OC_3H_6)_s \qquad (B)$$

$R^2$ étant un groupe alkyle ou alcényle en $C_{16-26}$,
r étant le nombre moyen de moles de $-(OC_2H_4)-$ par molécule de formule (B) présente dans le composé et étant compris entre 0,01 et 14,99,
s étant le nombre moyen de moles de $-(OC_3H_6)-$ par molécule de formule (B) présente dans le composé et étant compris entre 0,01 et 14,99, et

$$r + s \leq 15.$$

15. Utilisation d'un système d'émulsifiant selon la revendication 11 ou d'une composition pour stabiliser une émulsion, la composition comprenant :

a) un composé de type alcool gras alcoxylé de formule (A)

$$R^1(OC_2H_4)_n(OC_3H_6)_m \qquad (A)$$

$R^1$ étant un groupe alkyle ou alcényle en $C_{16-26}$,
n étant le nombre moyen de moles de $-(OC_2H_4)-$ par molécule de formule (A) présente dans le composé et étant compris entre 15 et 100,
m étant le nombre moyen de moles de $-(OC_3H_6)-$ par molécule de formule (A) présente dans le composé et étant compris entre 0 et 20,
et n + m > 15 ; et

b) un composé de type alcool gras alcoxylé de formule (B)

$$R^2(OC_2H_4)_r(OC_3H6)_s \qquad (B)$$

$R^2$ étant un groupe alkyle ou alcényle en $C_{16-26}$,
r étant le nombre moyen de moles de $-(OC_2H_4)-$ par molécule de formule (B) présente dans le composé et étant compris entre 0,01 et 14,99,
s étant le nombre moyen de moles de $-(OC_3H_6)-$ par molécule de formule (B) présente dans le composé et étant compris entre 0,01 et 14,99, et

$$r + s \leq 15.$$

**EP 3 664 768 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4122470